# DEMANDE DE BREVET EUROPEEN

(11) **EP 3 912 682 A1**
(43) Date de publication de la demande: **24.11.2021**
(21) Numéro de dépôt: 21183339.7
(22) Date de dépôt: 26.03.2014
(51) Int. Cl.: A61Q 5/00, A61Q 19/00, A61K 8/03, A61K 8/02, A61K 8/19, A61K 8/26, A61K 8/37, A61Q 1/00

(54) **COMPOSITIONS COSMÉTIQUES TRIPHASIQUES COMPRENANT DES NACRES**

(30) Priorité: 26.03.2013 FR 1352735; 26.03.2013 FR 1352727
(62) Demande divisionnaire de: 14713089.2
(71) Demandeur: L'OREAL, 75008 Paris (FR)
(72) Inventeur: ARNAUD, Pascal, 94240 L'HAY LES ROSES (FR); GINESTON, Sylvie, 94100 SAINT MAUR DES FOSSES (FR)
(74) Mandataire: Lavoix

(57) **Abrégé**

La présente invention concerne une composition cosmétique comprenant un milieu physiologiquement acceptable contenant une phase aqueuse, une phase huileuse et des particules de nacres, constituées d'un substrat enrobé, partiellement ou totalement, d'une ou plusieurs couches.

## Description

La présente invention a pour objet des compositions cosmétiques triphasiques, notamment pour le maquillage et/ou le soin, comprenant des nacres spécifiques. Plus particulièrement, la présente invention a pour objet des compositions comprenant une phase huileuse, une phase aqueuse et des particules de nacres se présentant sous la forme de trois couches superposées au repos.

Il est connu de l'homme du métier d'utiliser des nacres dans des produits de maquillage, de soin ou capillaires pour apporter des effets optiques de couleur et de brillance sur la peau, les lèvres, les cils, les ongles ou encore sur les cheveux.

Afin de mettre en valeur ces nacres, on peut simplement les disperser dans une phase liquide unique, transparente ou translucide qui présente un aspect visuel attrayant, caractérisé par le mouvement des nacres dans le milieu, lorsque l'on agite le produit.

Ces nacres peuvent ainsi être véhiculées dans une phase huileuse, car elles se dispersent bien dans ce milieu et elles ont peu tendance à s'agglomérer. Il existe ainsi sur le marché des produits constitués uniquement d'une phase huileuse liquide dans laquelle les nacres sont dispersées et qui ont pour fonction d'enluminer la peau ou les cheveux.

La quantité importante des huiles peut limiter toutefois l'effet coloriel après application sur la peau ou les cheveux surtout si les huiles ne sont pas volatiles et les propriétés sensorielles peuvent être dégradées en particulier en terme de fraicheur, de sensation grasse et de collant.

Pour éviter ces inconvénients, on peut véhiculer les nacres dans un milieu liquide aqueux, mais on observe dans ce cas une forte tendance à l'agglomération, qui se manifeste en particulier lorsque le produit est au repos. Il se forme alors une couche compacte issue de la sédimentation et du tassement des nacres, que l'on a du mal à disperser par agitation.

Par ailleurs, un simple milieu aqueux ne permet pas d'obtenir des propriétés sensorielles satisfaisantes en particulier en termes de propriétés d'application et de confort dans le temps.

De plus, lorsque le produit est de nouveau au repos, on observe le plus souvent qu'une partie des nacres adhèrent le long des parois du flacon au dessus du liquide, ce qui diminue son aspect esthétique.

Ce défaut est particulièrement visible lorsque le remplissage du flacon par le liquide n'est que partiel et que les parois du flacon sont en verre. Il est donc souhaitable de trouver des nacres qui ne présentent pas cet inconvénient et qui restent confinées dans le milieu liquide après agitation.

Il reste donc nécessaire de rechercher un moyen d'obtenir une dispersion de nacres dans un milieu liquide, transparent ou translucide, ne présentant pas un phénomène d'agglomération au repos, et qui conduit à un produit qui possède de bonnes propriétés d'application, de fraîcheur et de confort dans le temps.

Il reste donc également nécessaire de rechercher un moyen d'obtenir une dispersion de nacres dans un milieu liquide, transparent ou translucide, ne présentant pas un phénomène d'adhérence des nacres sur les parois dans le récipient contenant ladite dispersion, et qui conduit à un produit qui possède de bonnes propriétés d'application, de fraîcheur et de confort dans le temps.

La présente invention a donc pour but de fournir une composition cosmétique comprenant des nacres qui présente des propriétés d'application, de fraîcheur et de confort dans le temps satisfaisantes.

La présente invention a également pour but de fournir une composition cosmétique comprenant des nacres qui présente un aspect visuel attrayant.

La présente invention a également pour but de fournir une composition cosmétique comprenant des nacres qui possède des propriétés de réhomogénéisation satisfaisantes et qui présente au repos trois phases visuellement distinctes.

La présente invention a également pour but de fournir une composition cosmétique, transparente ou translucide, ne présentant pas de phénomène d'agglomération des nacres au repos.

La présente invention a également pour but de fournir une composition cosmétique, transparente ou translucide, comprenant des nacres qui sédimentent dans la phase aqueuse sans adhérer aux parois du récipient les contenant.

Ainsi, la présente invention concerne une composition cosmétique comprenant un milieu physiologiquement acceptable contenant :
(1) une phase aqueuse ;
(2) une phase huileuse de densité inférieure à celle de la phase aqueuse ; et
(3) des particules de nacres, dispersées dans la phase aqueuse, constituées d'un substrat enrobé, partiellement ou totalement, d'une ou plusieurs couches, l'une au moins des couches étant une couche d'oxyde métallique,
   lesdites particules de nacres étant choisies parmi les particules suivantes :
   - soit des particules de nacres (P1) dont la taille moyenne est comprise entre 2 µm et 1 000 µm, dans lesquelles le substrat est du mica ou de l'alumine,
      lesdites particules ne comprenant pas de couche de silice en surface, et
      sous réserve que, lorsque la couche d'oxyde métallique comprend plus de 30% en poids de dioxyde de titane par rapport au poids total des particules, la taille moyenne desdites particules est comprise entre 2 µm et 20 µm ;
   - soit des particules de nacres (P2) dans lesquelles le substrat est choisi dans le groupe constitué de la silice, du borosilicate, du mica et de l'alumine, et lorsque le substrat est du mica ou de l'alumine, les particules de nacres comprennent une couche de silice en surface, et
      lorsque l'oxyde métallique est un oxyde de fer, la teneur en oxyde de fer est inférieure à 50% en poids par rapport au poids total des particules,
      ladite composition cosmétique comprenant de 5% à 85% en poids de phase huileuse par rapport au poids total de ladite composition.

De manière inattendue, il a été trouvé par les inventeurs qu'il est possible d'obtenir un produit répondant aux critères recherchés concernant les propriétés d'application, de fraîcheur et de confort dans le temps et qui possède un aspect visuel attrayant, en dispersant des nacres spécifiques dans une phase aqueuse en présence d'une phase huileuse incompatible avec cette phase aqueuse.

Lorsque le produit est au repos, il se présente sous la forme de trois couches superposées distinctes, la phase huileuse constituant la couche supérieure, la phase aqueuse la couche intermédiaire et les nacres la couche inférieure. Sous agitation, les trois couches se mélangent intimement pour former un produit homogène que l'on peut alors appliquer.

Les compositions selon l'invention sont donc des compositions triphasiques au repos, dans lesquelles les nacres restent confinées dans le milieu liquide après agitation.

Selon un mode de réalisation, la présente invention concerne une composition cosmétique comprenant un milieu physiologiquement acceptable contenant :
(1) une phase aqueuse ;
(2) une phase huileuse de densité inférieure à celle de la phase aqueuse ; et
(3) des particules de nacres (P1), dispersées dans la phase aqueuse, constituées d'un substrat enrobé, partiellement ou totalement, d'une ou plusieurs couches, la taille moyenne desdites particules de nacres étant comprise entre 2 µm et 1 000 µm,
   ledit substrat étant du mica ou de l'alumine,
   l'une au moins des couches étant une couche d'oxyde métallique,
   lesdites particules (P1) ne comprenant pas de couche de silice en surface, et sous réserve que, lorsque la couche d'oxyde métallique comprend plus de 30% en poids de dioxyde de titane par rapport au poids total des particules, la taille moyenne desdites particules est comprise entre 2 µm et 20 µm,
ladite composition cosmétique comprenant de 5% à 70% en poids de phase huileuse par rapport au poids total de ladite composition.

Selon ce mode de réalisation, au repos, les phases aqueuse et huileuse se séparent progressivement et les nacres sédimentent dans la phase aqueuse pour former une couche que l'on peut facilement redisperser en agitant de nouveau le produit.

Selon un autre mode de réalisation, la présente invention concerne une composition cosmétique comprenant un milieu physiologiquement acceptable contenant :
(1) une phase aqueuse ;
(2) une phase huileuse de densité inférieure à celle de la phase aqueuse ; et
(3) des particules de nacres (P2), dispersées dans la phase aqueuse, constituées d'un substrat enrobé, partiellement ou totalement, d'une ou plusieurs couches,
   l'une au moins des couches étant une couche d'oxyde métallique, et ledit substrat étant choisi dans le groupe constitué de la silice, du borosilicate, du mica et de l'alumine,
   dans laquelle, lorsque le substrat est du mica ou de l'alumine, les particules de nacres (P2) comprennent une couche de silice en surface, et
   lorsque l'oxyde métallique est un oxyde de fer, la teneur en oxyde de fer est inférieure à 50% en poids par rapport au poids total des particules,
ladite composition cosmétique comprenant de 5% à 85% en poids de phase huileuse par rapport au poids total de ladite composition.

Selon ce mode de réalisation, au repos, les phases aqueuse et huileuse se séparent progressivement et les nacres sédimentent dans la phase aqueuse sans adhérer aux parois du flacon les contenant.

### Nacres

Les compositions cosmétiques selon l'invention comprennent des particules de nacres, dispersées dans la phase aqueuse, ces particules pouvant être identiques ou différentes. Les compositions selon l'invention peuvent donc comprendre des mélanges de particules de nacres de nature différente.

Dans le cadre de la présente invention, par « nacres », il faut comprendre des particules colorées de toute forme, irisées ou non, qui présentent un effet de couleur par interférence optique.

Ces particules de nacres se présentent sous la forme d'une dispersion de particules dans la phase aqueuse, et, comme indiqué ci-dessus, une fois la composition agitée, celles-ci forment avec les phases aqueuse et huileuse un produit homogène, facile à appliquer sur les surfaces kératiniques, notamment sur la peau ou les cheveux.

Selon un mode de réalisation, les nacres sont présentes dans la composition de l'invention en une quantité de matière active allant de 0,1% à 30% en poids, de préférence de 0,5% à 20% en poids et de manière encore plus préférentielle de 2% à 15% en poids, par rapport au poids total de ladite composition.

Les particules de nacres selon l'invention sont des particules composites, constituées de plusieurs matériaux. Les particules peuvent être de forme variée, par exemple sous forme plaquettaire et lamellaire.

Selon l'invention, les nacres présentent une structure multicouche constituée d'un substrat naturel ou synthétique.

Les particules de nacres utilisées dans le cadre de l'invention comprennent un substrat qui est enrobé par une ou plusieurs couches d'un matériau différent du substrat. Ces particules sont donc constituées de plusieurs matériaux.

Elles comprennent ainsi une couche de base correspondant au substrat sur laquelle se superpose au moins une couche d'un autre matériau. Le substrat selon l'invention peut être recouvert par exemple par une, deux ou trois couches distinctes de nature différente.

Le substrat des particules de nacres peut être enrobé partiellement par au moins une couche d'un autre matériau. Dans le cadre de la présente invention, par « enrobé partiellement », on entend que le substrat est recouvert par au moins une couche à raison de 50% à 99,9% de la surface dudit substrat.

Selon un mode de réalisation, le substrat des particules de nacres est enrobé totalement par au moins une couche d'un autre matériau. Ce mode de réalisation correspond au cas où la totalité de la surface externe du substrat, à savoir la surface enrobée par au moins une couche, est recouverte par ladite couche.

Dans le cadre de la présente invention, les particules de nacres sont définies par leur taille moyenne que l'on mesure par granulométrie. La taille moyenne des nacres correspond à leur diamètre apparent car elles sont assimilées, lors de la mesure, à des sphères. La taille moyenne est caractérisée par la D₅₀ qui correspond à la granulométrie moyenne de la moitié de la population.

La mesure de la granulométrie s'effectue de la manière suivante :
On introduit dans un flacon 0,1 g de nacre dans 12 à 14 g de myristate d'isopropyle. On agite l'échantillon jusqu'à obtenir une dispersion complète puis on mesure la granulométrie des nacres dans ce milieu à l'aide du Mastersizer 2000 de la société Malvern.

Selon un mode de réalisation, les particules de nacres de l'invention (P1) peuvent comprendre du dioxyde de titane à titre d'oxyde métallique. Selon ce mode de réalisation, lorsque le taux de dioxyde de titane est inférieur ou égal à 30%, les particules de nacres ont une taille moyenne qui peut varier de 2 µm à 1 000 µm, de préférence de 3 µm à 500 µm et de manière encore plus préférentielle de 5 µm à 250 µm.

Selon ce mode de réalisation, lorsque le taux de dioxyde de titane est supérieur à 30%, les particules de nacres (P1) ont une taille moyenne qui peut varier de 2 µm à 20 µm, de préférence de 3 µm à 15 µm et de manière encore plus préférentielle de 5 µm à 12 µm.

Les taux mentionnés pour le dioxyde de titane correspondent à des pourcentages en poids de dioxyde de titane par rapport au poids total des particules de nacres.

Selon ce mode de réalisation, les particules de nacres (P1) sont constituées de mica ou d'alumine à titre de substrat. De préférence, ce substrat est du mica, naturel ou synthétique.

Les particules de nacres (P2) selon l'invention ont de préférence une taille moyenne qui peut varier de 2 µm à 1 000 µm, de préférence de 3 µm à 500 µm et de manière encore plus préférentielle de 5 µm à 250 µm.

Selon un mode de réalisation, les particules de nacres (P2) de l'invention peuvent comprendre de l'oxyde de fer à titre d'oxyde métallique. Selon l'invention, lorsque le substrat des particules (P2) est enrobé par une ou plusieurs couches d'oxyde de fer, le taux global de ces couches doit être inférieur ou égal à 50%.

Selon un mode de réalisation, le substrat des particules de nacres (P2) selon l'invention est un substrat synthétique choisi parmi la silice ou le borosilicate. De préférence, le substrat est du borosilicate.

Selon un autre mode de réalisation, le substrat des particules de nacres (P2) selon l'invention est un substrat naturel ou synthétique choisi parmi le mica et l'alumine. Selon ce mode de réalisation, les particules de nacres (P2) comprennent alors nécessairement de la silice comme couche la plus externe.

Ainsi, lorsque le substrat des particules (P2) est du mica ou de l'alumine, les particules (P2) comprennent une couche de silice et cette couche de silice se trouve en surface desdites particules. Il s'agit alors de la couche la plus externe desdites particules, c'est-à-dire que cette couche de silice ne doit pas être recouverte d'une autre couche.

Parmi les couches recouvrant le substrat des particules (P1) ou (P2), l'une au moins est une couche d'oxyde métallique. Selon un mode de réalisation, les oxydes métalliques sont choisis dans le groupe constitué de l'oxyde de titane, des oxydes de fer, des oxydes de chrome, des oxydes d'étain, des oxydes d'alumine et de leurs mélanges.

Ainsi, le substrat est enrobé au moins partiellement d'au moins une couche choisie par exemple parmi l'oxyde de titane, les oxydes de fer notamment Fe₂O₃, d'étain, de chrome, et l'alumine.

Selon un mode de réalisation, le substrat des particules (P1) ou (P2) est enrobé d'une couche unique d'oxyde métallique ou de plusieurs couches distinctes d'oxyde métallique.

En particulier, les particules de nacres (P1) ou (P2) selon l'invention sont constituées du substrat recouvert d'une couche unique d'oxyde métallique, ou de deux ou trois couches différentes d'oxyde métallique.

Selon un mode de réalisation, les particules de nacres (P1) selon l'invention comprennent au moins une couche d'oxyde de fer.

Selon un mode de réalisation, les particules de nacres (P1) selon l'invention comprennent au moins une couche d'oxyde de titane.

Selon un mode de réalisation, les particules de nacres (P1) selon l'invention comprennent au moins une couche d'oxyde de fer et au moins une couche d'oxyde de titane.

Selon l'invention, le substrat des particules (P1) ou (P2) peut également être enrobé en outre par au moins une couche constituée d'oxychlorure de bismuth, de bleu d'outremer, de bleu de Prusse, de violet de manganèse, de carmin de cochenille et de leurs mélanges.

Les particules selon l'invention (P1) ou (P2) peuvent donc comprendre, outre au moins une couche d'oxyde métallique, une couche supplémentaire telle que définie ci-dessus.

Les particules (P1) selon l'invention peuvent également comprendre en outre au moins une couche de silice. Selon ce mode de réalisation, cette couche de silice ne doit pas se trouver en surface desdites particules. Elle ne doit donc pas être la couche la plus externe desdites particules. Si les particules de nacres (P1) selon l'invention comprennent une couche de silice, celle-ci doit être recouverte d'une autre couche par exemple d'une couche d'oxyde métallique.

A titre illustratif de nacres (P1) pouvant être mises en œuvre dans le cadre de la présente invention, on peut notamment citer les nacres commercialisées par la société BASF Personal Care Ingredients dans les gammes : Flamenco, Timica, Cloisonne, Cosmica, Gemtone et Chroma-lite ; les nacres commercialisées par la société MERCK dans les gammes : Timiron, Colorona et Microna et les nacres commercialisées par la société SUDARSHAN CHEMICAL dans la gamme Pearlescent Pigment Prestige.

Selon un mode de réalisation, les particules de nacres (P2) selon l'invention comprennent de la silice ou du borosilicate comme substrat et au moins deux couches d'oxyde métallique, et de préférence une couche d'oxyde de titane et une couche d'oxyde d'étain.

Selon un mode de réalisation, les particules de nacres (P2) selon l'invention comprennent du mica à titre de substrat.

Selon un mode de réalisation, les particules de nacres (P2) selon l'invention comprennent du mica à titre de substrat, une ou deux couches d'oxyde métallique et de la silice comme couche externe.

A titre illustratif de nacres (P2) pouvant être mises en oeuvre dans le cadre de la présente invention, on peut notamment citer les nacres commercialisées par la société BASF Personal Care Ingredients sous le nom de Reflecks Gilded Gold G232L ; les nacres commercialisées par la société MERCK sous le nom de Timiron Splendid Red et Timiron Artic Silver, sous le nom de Colorona Precious Gold, Xirona Magic Mauve, et Ronastar Gold, et les nacres commercialisées par la société NIPPON SHEET GLASS sous le nom de Metashine MC 1080RR et Microglass Metashine MC 2080 PSS1.

### Phase aqueuse

Les compositions cosmétiques selon l'invention comprennent une phase aqueuse dans laquelle les particules de nacres sont dispersées.

Selon un mode de réalisation, la phase aqueuse comprend de 5% à 80%, notamment de 10% à 80%, en poids d'eau par rapport au poids total de la composition.

Plus particulièrement, cette phase aqueuse peut comprendre de 10% à 60%, notamment de 15% à 60%, en poids d'eau par rapport au poids total de la composition.

Selon un mode de réalisation, lorsque les particules de nacres sont des particules (P1) selon l'invention, la phase aqueuse comprend de 5% à 80%, de préférence de 10% à 60%, en poids d'eau par rapport au poids total de la composition.

Selon un mode de réalisation, lorsque les particules de nacres sont des particules (P2) selon l'invention, la phase aqueuse comprend de 10% à 80%, de préférence de 15% à 60%, en poids d'eau par rapport au poids total de la composition.

Une eau convenant à l'invention peut être aussi une eau de source naturelle ou une eau florale. En particulier, une eau convenant à l'invention peut être une eau florale telle que l'eau de bleuet et/ou une eau minérale telle que l'eau de VITTEL, l'eau de LUCAS ou l'eau de LA ROCHE POSAY et/ou une eau thermale.

Selon un mode de réalisation, la phase aqueuse des compositions de l'invention peut comprendre en outre au moins un solvant organique miscible à l'eau.

Ce solvant organique, miscible à l'eau, peut être choisi parmi :
- les monoalcools comprenant de 1 à 8 atomes de carbone, et notamment de 2 à 5 atomes de carbone, comme l'éthanol et l'isopropanol,
- les glycols comprenant de 2 à 8 atomes de carbone, comme le propylène glycol, le butylène glycol, le pentylène glycol, l'héxylène glycol, le dipropylène glycol et le caprylyl glycol,
- les polyéthylènes glycols,
- les alcools polyhydriques comprenant de 2 à 8 atomes de carbone comme le glycérol, et
- les mélanges de ceux-ci.

En particulier, la phase aqueuse des compositions de l'invention peut comprendre de l'éthanol, du propylène glycol, du glycérol, ou un mélange de ceux-ci. Selon un mode de réalisation, la phase aqueuse comprend, outre de l'eau, un mélange d'éthanol, de propylène glycol et de glycérol.

La phase aqueuse peut aussi contenir tout composé hydrosoluble ou hydrodispersible compatible avec une phase aqueuse. Parmi ces composés, on peut citer les actifs, les colorants, les sels comme le chlorure de sodium et le sulfate de magnésium, les gélifiants, les conservateurs et tout autre additif hydrosoluble utilisé communément dans les produits cosmétiques.

### Phase huileuse

Les compositions selon l'invention comprennent également une phase huileuse dont la densité est inférieure à celle de la phase aqueuse. Ainsi, au repos, la phase huileuse est au-dessus de la phase aqueuse et constitue la couche supérieure de la composition selon l'invention.

La densité selon l'invention correspond à la densité à la température ambiante.

Selon un mode de réalisation, la phase huileuse représente de 5% à 85%, notamment de 5% à 70%, de préférence de 20% à 70%, notamment de 20% à 60%, et préférentiellement de 30% à 60%, notamment de 30% à 50%, en poids par rapport au poids total de ladite composition.

Selon un mode de réalisation, lorsque les particules de nacres sont des particules (P1) selon l'invention, la phase huileuse représente de 5% à 70%, de préférence de 20% à 60%, et préférentiellement de 30% à 50%, en poids par rapport au poids total de la composition.

Selon un mode de réalisation, lorsque les particules de nacres sont des particules (P2) selon l'invention, la phase huileuse représente de 5% à 85%, de préférence de 20% à 70%, et préférentiellement de 30% à 60%, en poids par rapport au poids total de la composition.

La phase huileuse (ou phase grasse) des compositions selon l'invention comprend au moins une huile. Elle peut être constituée d'une huile unique ou d'un mélange de plusieurs huiles. On entend par huile, tout corps gras sous forme liquide à température ambiante (20-25°C) et à pression atmosphérique. Ces huiles peuvent être d'origine animale, végétale, minérale ou synthétique.

Selon un mode de réalisation, les huiles sont choisies dans le groupe constitué des huiles hydrocarbonées, des huiles siliconées, des huiles fluorées et de leurs mélanges.

Au sens de la présente invention, on entend par « huile hydrocarbonée », une huile contenant principalement des atomes d'hydrogène et de carbone.

On entend par « huile siliconée », une huile comprenant au moins un atome de silicium, et notamment au moins un groupe Si-O.

On entend par « huile fluorée », une huile comprenant au moins un atome de fluor.

Les huiles peuvent éventuellement comprendre des atomes d'oxygène, d'azote, de soufre et/ou de phosphore, par exemple, sous la forme de radicaux hydroxyles ou acide.

Selon un mode de réalisation, la phase huileuse des compositions de l'invention comprend au moins une huile volatile et/ou au moins une huile non volatile.

### Huiles volatiles

Selon un mode de réalisation, la phase huileuse des compositions de l'invention comprend au moins une huile volatile. La phase huileuse des compositions de l'invention peut comprendre un mélange de plusieurs huiles volatiles.

Par « huile volatile », on entend tout milieu non aqueux susceptible de s'évaporer de la peau ou des lèvres, en moins d'une heure, à température ambiante et pression atmosphérique. L'huile volatile est une huile cosmétique volatile, liquide à température ambiante. Plus précisément, une huile volatile présente une vitesse d'évaporation comprise entre 0,01 et 200 mg/cm²/min, bornes incluses.

Pour mesurer cette vitesse d'évaporation on introduit dans un cristallisoir, de diamètre 7 cm, placée sur une balance se trouvant dans une grande enceinte d'environ 0,3 m³ régulée en température, à une température de 25°C, et en hygrométrie, à une humidité relative de 50%, 15 g d'huile ou de mélange d'huile à tester. On laisse le liquide s'évaporer librement, sans l'agiter, en assurant une ventilation par un ventilateur (PAPST-MOTOREN, référence 8550 N, tournant à 2 700 tours par minute) disposé en position verticale au-dessus du cristallisoir contenant ladite huile ou ledit mélange, les pales étant dirigées vers le cristallisoir et à une distance de 20 cm par rapport au fond du cristallisoir. On mesure à intervalles réguliers la masse d'huile restant dans le cristallisoir. Les vitesses d'évaporation sont exprimées en mg d'huile évaporée par unité de surface (cm²) et par unité de temps (minute).

Les huiles volatiles peuvent être hydrocarbonées, siliconées ou fluorées.

Les huiles volatiles peuvent être choisies parmi les huiles hydrocarbonées ayant de 8 à 16 atomes de carbone, et notamment les alcanes ramifiés en C₈-C₁₆ (appelées aussi isoparaffines ou isoalcanes), comme l'isododécane (encore appelé 2,2,4,4,6-pentaméthylheptane), l'isodécane, l'isohexadécane, et, par exemple, les huiles vendues sous les noms commerciaux d'ISOPARS® ou de PERMETHYLS®.

Comme huile volatile hydrocarbonée, on peut également citer les alcanes linéaires en C₉-C₁₇, comme le dodécane (C₁₂) et de tétradécane (C₁₄), commercialisés respectivement sous les références de PARAFOL® 12-97 et PARAFOL® 14-97 (Sasol) et comme les alcanes obtenus suivant le procédé décrit dans la demande internationale WO2007/068371 A1, tels que le mélange d'undécane (C₁₁) et de tridécane (C₁₃) commercialisé sous la référence de CETIOL® UT (Cognis).

Comme huiles volatiles siliconées, on peut aussi utiliser les silicones volatiles, comme, par exemple, les huiles de silicones linéaires ou cycliques volatiles, notamment, celles ayant une viscosité inférieure ou égale à 8 centistokes (cst) (8 x 10⁻⁶ m²/s), et ayant, notamment, de 2 à 10 atomes de silicium, et en particulier, de 2 à 7 atomes de silicium, ces silicones comportant, éventuellement, des groupes alkyle ou alcoxyle ayant de 1 à 10 atomes de carbone. Comme huile de silicone volatile utilisable dans l'invention, on peut citer, notamment, les diméthicones de viscosité 5 et 6 cst, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

Plus particulièrement, comme huile volatile siliconée, on peut citer les huiles de silicone linéaires ou cycliques ayant de 2 à 7 atomes de silicium, ces silicones comportant éventuellement des groupes alkyles ou alcoxyles ayant de 1 à 10 atomes de carbone.

Parmi les huiles volatiles siliconées, le dodecaméthyl pentasiloxane est préféré.

Comme huile volatile fluorée, on peut citer par exemple le nonafluorométhoxybutane ou le perfluorométhylcyclopentane, et leurs mélanges.

### Huiles non volatiles

Selon un mode de réalisation, la phase huileuse des compositions de l'invention comprend au moins une huile non volatile. La phase huileuse des compositions de l'invention peut comprendre un mélange de plusieurs huiles non volatiles.

Par « huile non volatile », on entend une huile restant sur la peau ou la fibre kératinique à température ambiante et pression atmosphérique. Plus précisément, une huile non volatile présente une vitesse d'évaporation strictement inférieure à 0,01 mg/cm²/min.

Les huiles non volatiles peuvent, notamment, être choisies parmi les huiles hydrocarbonées, fluorées et/ou les huiles siliconées non volatiles.

Comme huile hydrocarbonée non volatile, on peut notamment citer :
- les huiles hydrocarbonées d'origine animale, comme le perhydrosqualène,
- les huiles hydrocarbonées d'origine végétale, telles que les esters de phytostéaryle, tels que l'oléate de phytostéaryle, l'isostéarate de physostéaryle et le glutamate de lauroyl/octyldodécyle/phytostéaryle (AJINOMOTO, ELDEW PS203), les triglycérides constitués d'esters d'acides gras et de glycérol, en particulier, dont les acides gras peuvent avoir des longueurs de chaînes variant de C₄ à C₃₆, et, notamment, de C₁₈ à C₃₆, ces huiles pouvant être linéaires ou ramifiées, saturées ou insaturées ; ces huiles peuvent, notamment, être des triglycérides heptanoïques ou octanoïques, l'huile de karité, de luzerne, de pavot, de potimarron, de millet, d'orge, de quinoa, de seigle, de bancoulier, de passiflore, le beurre de karité, l'huile d'aloès, l'huile d'amande douce, l'huile d'amande de pêche, l'huile d'arachide, l'huile d'argan, l'huile d'avocat, l'huile de baobab, l'huile de bourrache, l'huile de brocoli, l'huile de calendula, l'huile de caméline, l'huile de canola, l'huile de carotte, l'huile de carthame, l'huile de chanvre, l'huile de colza, l'huile de coton, l'huile de coprah, l'huile de graine de courge, l'huile de germe de blé, l'huile de jojoba, l'huile de lys, l'huile de macadamia, l'huile de maïs, l'huile de perle de prairie ('meadowfoam'), l'huile de millepertuis, l'huile de monoï, l'huile de noisette, l'huile de noyaux d'abricot, l'huile de noix, l'huile d'olive, l'huile d'onagre, l'huile de palme, l'huile de pépins de cassis, l'huile de pépins de kiwi, l'huile de pépins de raisin, l'huile de pistache, l'huile de potimarron, l'huile de potiron, l'huile de rosier muscat, l'huile de sésame, l'huile de soja, l'huile de tournesol, l'huile de ricin, et l'huile de graines de melon d'eau, et leurs mélanges, ou encore des triglycérides d'acides caprylique/caprique, comme ceux vendus par la société STEARINERIES DUBOIS ou ceux vendus sous les dénominations MIGLYOL 810®, 812® et 818® par la société DYNAMIT NOBEL,
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam ;
- les éthers de synthèse ayant de 10 à 40 atomes de carbone ;
- les esters de synthèse, comme les huiles de formule R₁COOR₂, dans laquelle R₁ représente un reste d'un acide gras linéaire ou ramifié comportant de 1 à 40 atomes de carbone, et R₂ représente une chaîne hydrocarbonée, notamment, ramifiée contenant de 1 à 40 atomes de carbone à condition que la somme du nombre d'atomes de carbone des chaînes R₁ et R₂ soit supérieure ou égale 10. Les esters peuvent être, notamment, choisis parmi les esters d'alcool et d'acide gras, comme par exemple : l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés, comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools, comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diéthyl 2-d'hexanoate de propylèneglycol, et leurs mélanges, les benzoates d'alcools en C₁₂-C₁₅, le laurate d'hexyle, les esters de l'acide néopentanoïque, comme le néopentanoate d'isodécyle, le néopentanoate d'isotridécyle, le néopentanoate d'isostéaryle, le néopentanoate d'octyldocécyle, les esters de l'acide isononanoïque, comme l'isononanoate d'isononyle, l'isononanoate d'isotridécyle, l'isononanoate d'octyle, les esters hydroxylés comme le lactate d'isostéaryle, le malate de di-isostéaryle ;
- les alcools gras liquides à température ambiante à chaîne carbonée ramifiée et/ou insaturée ayant de 12 à 26 atomes de carbone, comme le 2-octyldodécanol, l'alcool isostéarylique, l'alcool oléique, le 2-hexyldécanol, le 2-butyloctanol, et le 2-undécylpentadécanol ;
- les acides gras supérieurs en C₁₂-C₂₂, tels que l'acide oléique, l'acide linoléique, l'acide linolénique, et leurs mélanges ;
- les carbonates de di-alkyle, les 2 chaînes alkyles pouvant être identiques ou différentes, tels que le dicaprylyl carbonate commercialisé sous la dénomination CETIOL CC®, par COGNIS ;
- les huiles fluorées éventuellement partiellement hydrocarbonées et/ou siliconées comme les huiles fluorosiliconées, les polyéthers fluorés, les silicones fluorées telles que décrit dans le document EP-A-847 752 ;
- les huiles siliconées comme les polydiméthylsiloxanes (PDMS) non volatiles, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates ; et
- leurs mélanges.

Parmi les hydrocarbures linéaires ou ramifiés, d'origine minérale ou synthétique, on utilise de préférence les huiles de paraffines ou l'huile de vaseline.

Parmi les huiles hydrocarbonées d'origine végétale, on peut citer de préférence les huiles végétales, comme l'huile d'amande douce, l'huile de jojoba ou l'huile de macadamia.

Selon un mode de réalisation, la phase huileuse comprend au moins une huile hydrocarbonée non volatile. De préférence, cette huile hydrocarbonée non volatile est choisie parmi les alcools gras liquides à température ambiante et les esters de synthèse tels que définis ci-dessus, ainsi que leurs mélanges.

Selon un mode de réalisation, la phase huileuse comprend de l'isononanoate d'isononyle.

Selon un mode de réalisation, la phase huileuse comprend de l'octyldodécanol.

Selon un mode de réalisation, la phase huileuse comprend au moins une huile siliconée volatile. De préférence, cette huile siliconée volatile est le dodécaméthylpentasiloxane.

La phase huileuse est de préférence limpide et transparente.

La phase huileuse peut aussi contenir tout composé liposoluble. Parmi ces composés liposolubles, on peut citer les parfums, les colorants, les actifs, ou les gélifiants.

### Milieu physiologiquement acceptable

Outre les composés indiqués précédemment, à savoir la phase aqueuse, la phase huileuse et les particules de nacres, une composition selon l'invention comprend un milieu physiologiquement acceptable.

Par "milieu physiologiquement acceptable", on entend désigner un milieu convenant particulièrement à l'application d'une composition de l'invention sur la peau, les cheveux ou les lèvres.

Le milieu physiologiquement acceptable est généralement adapté à la nature du support sur lequel doit être appliquée la composition, ainsi qu'à l'aspect sous lequel la composition doit être conditionnée.

### Matières colorantes

Les compositions de l'invention peuvent également comprendre, en plus des particules de nacres, une matière colorante additionnelle ou un mélange de matières colorantes additionnelles.

Selon un mode préféré, la teneur en matières colorantes additionnelles va de 0% à 1% en poids, en particulier de 0,0001% à 0,5% en poids, par rapport au poids total de ladite composition.

Selon un mode de réalisation, les compositions selon l'invention comprennent au moins un colorant. Elles peuvent comprendre un colorant ou un mélange de plusieurs colorants, ces colorants pouvant être présents dans la phase aqueuse et/ou huileuse.

Selon un mode de réalisation, les compositions selon l'invention comprennent un colorant dans la phase aqueuse et un autre colorant dans la phase huileuse.

Par "colorants", il faut comprendre des composés généralement organiques solubles dans les corps gras comme les huiles ou dans une phase hydroalcoolique.

La composition cosmétique selon l'invention peut donc comprendre également des colorants hydrosolubles ou liposolubles.

Les colorants liposolubles sont, par exemple, le rouge Soudan, le DC Red 21, le DC Red 27, le DC Green 6, le β-carotène, l'huile de soja, le brun Soudan, le DC Yellow 11, le DC Violet 2, le DC orange 5 et le jaune quinoléine.

Les colorants hydrosolubles sont, par exemple, le jus de betterave et le caramel, ou encore les colorants DC Red 4, DC Red 6, DC Red 22, DC Red 28, DC Red 30, DC Red 33, DC Orange 4, FDC Yellow 5, FDC Yellow 6, DC Yellow 8, FDC Green 3, DC Green 5 et le FDC Blue 1.

### Additifs

Une composition cosmétique selon l'invention peut également comprendre en outre tout additif usuellement utilisé dans le domaine concerné, par exemple choisi parmi des gommes, des agents tensioactifs anioniques, cationiques, amphotères ou non ioniques, des agents tensioactifs silicones, des gommes, des résines, des agents dispersants, des polymères semi-cristallins, des agent antioxydants, des huiles essentielles, des conservateurs, des parfums, des neutralisants, des agents antiseptiques, des agents protecteurs contre les UV, des actifs cosmétiques, comme des vitamines, des agents hydratants, émollients ou protecteurs de collagène, et leurs mélanges.

Il relève des opérations de routine de l'homme du métier d'ajuster la nature et la quantité des additifs présents dans les compositions conformes à l'invention, de telle sorte que les propriétés cosmétiques et les propriétés de stabilité désirées de celles-ci n'en soient pas affectées.

### Applications

Les compositions cosmétiques couvertes par l'invention peuvent être des produits de soin, de maquillage du visage ou du corps ou encore des produits capillaires.

Ces compositions sont donc destinées à être appliquées sur la peau ou les cheveux.

Les compositions de l'invention peuvent être conditionnées dans des flacons de toute nature et de toutes formes communément utilisés pour les produits cosmétiques et plus préférentiellement dans des flacons transparents en verre ou matière plastique comme le polyéthylène téréphtalate (PET).

L'invention est particulièrement bien adaptée à des flacons dont les parois sont en verre.

La présente invention concerne également une méthode de traitement cosmétique non thérapeutique de la peau comprenant une étape d'application sur la peau d'au moins une couche d'une composition selon l'invention.

La présente invention concerne également un procédé non thérapeutique de maquillage et/ou de soin de la peau comprenant une étape d'application sur la peau d'au moins une couche d'une composition selon l'invention.

La présente invention concerne également un procédé de revêtement des fibres kératiniques comprenant une étape d'application sur lesdites fibres kératiniques d'une composition cosmétique selon l'invention.

Dans toute la demande, le libellé "comprenant un" ou "comportant un" signifie "comprenant au moins un" ou "comportant au moins" un sauf si le contraire est spécifié.

Dans toute la description ci-dessus, sauf mention contraire, le terme « compris(e) entre x et y » correspond à une gamme inclusive, c'est-à-dire que les valeurs x et y sont incluses dans la gamme.

### EXEMPLES

### Test d'évaluation de l'agglomération des nacres au repos

Afin d'évaluer l'agglomération des nacres au repos, le test suivant est effectué dans le cadre de la présente invention :
On introduit un échantillon de 20 g de la composition dans un flacon d'environ 30 mL (diamètre 2,5 cm, hauteur 8 cm).

Afin de garantir que l'échantillon prélevé est fidèle à la composition, on le conditionne après avoir homogénéisé la composition sous agitation. On peut ainsi prélever l'échantillon juste après la fabrication.

Le flacon est laissé au repos pendant 24 heures en position verticale, puis il est agité 10 fois par des mouvements énergiques et verticaux.

On juge ensuite l'agglomération des nacres :
- S'il y a une ré-homogénéisation totale des nacres dans l'ensemble de la composition, celle ci est conforme à l'invention,
- Si les nacres restent agglomérées au fond du flacon, ou si l'homogénéisation n'est que partielle alors la composition n'est pas conforme à l'invention.

### Exemples 1 à 3 : Influence de la nature du substrat de la nacre

Les exemples 1 à 3 permettent de montrer l'influence de la nature du substrat de la nacre (particules (P1)) sur le phénomène d'agglomération au repos.

| | | **% massique** |
|---|---|---|
| A | Eau | 10,50 |
| | Ethanol absolu | 16,8998 |
| | Propylène glycol | 25,20 |
| | Glycérol | 16,90 |
| | D&C Red N°4 | 0,0001 |
| B | Isononyl isononanoate | 20,00 |
| | D&C Violet N°2 | 0,0001 |
| **C** | **Nacre*** | **10,50** |
| | TOTAL | 100% |

| | | |
|---|---|---|
| * La nature de la nacre est mentionnée dans le tableau plus loin | | |

### Mode opératoire

Les compositions des exemples 1 à 3 sont préparées selon le protocole décrit ci-après.

On prépare séparément la phase aqueuse A en pesant les constituants dans un bécher puis en agitant au barreau aimanté jusqu'à homogénéisation.

On prépare séparément, de la même manière, la phase huileuse B.

On introduit ensuite la phase aqueuse A dans le bécher principal que l'on agite à l'aide d'une turbine défloculeuse-Raynerie à une vitesse de 500 tours / min.

La nacre (phase C) est ensuite introduite dans la phase aqueuse sous agitation de manière à l'homogénéiser correctement.

Puis la phase huileuse B est introduite dans le bécher en maintenant sous agitation.

On agite encore pendant 15 min la composition avant conditionnement.

| **Exemples** | **Nacres** | **Structure** |
|---|---|---|
| Exemple 1 (Invention) (particules (P1)) | Mica-oxyde de titane-oxyde de fer brun vendu sous la référence Pearlescent Pigment Flonac MX 30 C par la société Sudarshan Chemical | Mica/TiO₂/Ox.fer (82/14/4) |
| Exemple 2 (invention) (particules (P2)) | Silice- oxyde de titane-oxyde d'étain vendu sous la référence Xirona Magic Mauve par la société Merck | SiO₂/TiO₂/SnO₂ (88/10/2) |
| Exemple 3 (invention) (particules (P2)) | Calcium aluminium borosilicate- oxyde de titane-oxyde d'étain vendu sous la référence Metashine MC1080RR par la société Nippon Sheet Glass | Borosilicate/TiO₂/SnO₂ (78/21,5/0,5) |

On a donc constaté que des particules de nacres (P1) comme décrites ci-dessus dans la description permettent d'obtenir une composition conforme à l'invention, c'est-à-dire une composition triphasique au repos. De plus, dans ces compositions, les nacres sont facilement ré-homogénéisées après agitation.

Lorsque le substrat est différent de celui des particules (P1) de l'invention, cette homogénéisation des nacres n'est pas obtenue de façon aussi satisfaisante.

Les particules (P2) permettent d'obtenir des compositions triphasiques mais les compositions obtenues sont moins avantageuses en termes de propriétés de ré-homogénéisation des nacres. Ces particules permettent cependant d'obtenir des compositions avantageuses en termes d'adhésion aux parois (cf. exemples 15 et 16).

### Exemples 4 à 9 : Variation de la taille

Les exemples 4 à 9 illustrent l'invention pour des nacres (P1) qui contiennent un pourcentage de dioxyde de titane inférieur ou égal à 30%.

| | | **% massique** |
|---|---|---|
| A | Eau | 10,50 |
| | Ethanol absolu | 16,8998 |
| | Propylène glycol | 25,20 |
| | Glycérol | 16,90 |
| | D&C Red N°4 | 0,0001 |
| B | Isononyl isononanoate | 20,00 |
| | D&C Violet N°2 | 0,0001 |
| **C** | **Nacre** | **10,50** |
| | TOTAL | 100% |

| Exemples | **Nacres** | **Structure** | **Taille moyenne** | **%TiO₂** | **Aspect** |
|---|---|---|---|---|---|
| Exemple 4 (Invention) | Mica-oxyde de fer brun vendu sous la référence Pearlescent Pigment Prestige soft bronze par la société Sudarshan Chemical | Mica/Ox.fer (43/57) | 8 | 0 | Homogénéisation facile des nacres après agitation |
| Exemple 5 (Invention) | Mica-oxyde de titane-oxyde de fer brun vendu sous la référence Pearlescent Pigment Prestige soft beige par la société Sudarshan Chemical | Mica/TiO₂/Ox.fer/SnO₂ (41,8/20,2/37,8/0,20) | 8 | 20,2 | Homogénéisation facile des nacres après agitation |
| Exemple 6 (Invention) | Mica synthétique (fluorphlogopite)-oxyde de fer brun vendu sous la référence Sunshine Glitter Russet par la société Sun Chemical | Mica synthétique/Ox.fer (54,5/45,5) | 45 | 0 | Homogénéisation facile des nacres après agitation |
| Exemple 7 (Invention) | Mica-oxyde de fer brun vendu sous la référence Pearlescent Pigment Flonac MX 30 C par la société Sudarshan Chemical | Mica/Ox.fer (83/17) | 80 | 0 | Homogénéisation facile des nacres après agitation |
| Exemple 8 (Invention) | Mica-oxyde de titane-oxyde de fer brun vendu sous la référence Pearlescent Pigment Flonac MX 30 C par la société Sudarshan Chemical | Mica/TiO₂/Ox.fer (82/14/4) | 97 | 14,0 | Homogénéisation facile des nacres après agitation |
| Exemple 9 (Invention) | Mica synthétique (fluorphlogopite)-oxyde de titane-oxyde-oxyde de fer vendu sous la référence Sunshine Ultra Glitter Golden par la société Sun Chemical | Mica synthétique/TiO₂/O_{X}.fer (82/14,5/3,5) | 230 | 14,5 | Homogénéisation facile des nacres après agitation |

### Mode opératoire

Les compositions des exemples 4 à 9 sont préparées selon le protocole décrit ci-dessus pour les exemples 1 à 3.

### Evaluation sensorielle

Il a été demandé à un panel de 4 femmes, âgées de 25 à 50 ans, de maquiller l'exemple 4 sur le visage et le buste.

Il est ressorti de cette évaluation que le produit procure un résultat maquillage plaisant car peu nacré, le toucher est non gras et non collant.

### Exemples 10 à 12 : Influence du pourcentage de titane

Les exemples 10 à 12 permettent de montrer l'influence de la taille de la nacre (particules (P1)) lorsque le pourcentage d'oxyde de titane est supérieur à 30%.

| | | **% massique** |
|---|---|---|
| A | Eau | 10,50 |
| | Ethanol absolu | 16,8998 |
| | Propylène glycol | 25,20 |
| | Glycérol | 16,90 |
| | D&C Red N°4 | 0,0001 |
| B | Isononyl isononanoate | 20,00 |
| | D&C Violet N°2 | 0,0001 |
| **C** | **Nacre** | **10,50** |
| | TOTAL | 100% |

| Exemples | **Nacres** | **Structure** | **Taille moyenne** | **%TiO₂** | **Aspect** |
|---|---|---|---|---|---|
| Exemple 10 (Invention) | Mica-oxyde de titane vendu sous la référence Timiron silk green par la société Merck | Mica/riO₂/SnO₂ (31/68/1) | 12 | 68,0 | Homogénéisation facile des nacres après agitation |
| Exemple 11 (Comparatif) | Mica-oxyde de titane-oxyde d'étain vendu sous la référence Flamenco Summit Red R30D par la société BASF | Mica/riO₂/SnO₂ (55,9/43,6/0,50) | 22 | 43,6 | Forte agglomération des nacres, homogénéisation impossible |
| Exemple 12 (Comparatif) | Mica-oxyde de titane vendu sous la référence Flamenco Sparkle Green 820 J par la société BASF | Mica/TiO₂ (64/36) | 45 | 36,0 | Forte agglomération des nacres, homogénéisation impossible |

### Mode opératoire

Les compositions des exemples 10 à 12 sont préparées selon le protocole décrit ci-dessus pour les exemples 1 à 3.

On a donc constaté que des particules de nacres comme décrites ci-dessus dans la description (contenant plus de 30% en poids de dioxyde de titane mais de taille inférieure à 20 µm) permettent d'obtenir une composition conforme à l'invention, c'est-à-dire une composition triphasique au repos dans laquelle les nacres sont facilement ré-homogénéisées après agitation.

Lorsque la teneur en dioxyde de titane est supérieure à 30% en poids et la taille des particules est supérieure à 20 µm, cette homogénéisation des nacres n'est pas obtenue de façon satisfaisante.

### Exemples 13 et 14 : Variation de la nature de l'huile

Les exemples 13 et 14 illustrent l'invention pour différentes huiles.

| | | **% massique** |
|---|---|---|
| A | Eau | 10,50 |
| | Ethanol absolu | 16,8998 |
| | Propylène glycol | 25,20 |
| | Glycérol | 16,90 |
| | D&C Red N°4 | 0,0001 |
| B | **Huile** | **20,00** |
| | D&C Violet N°2 | 0,0001 |
| **C** | Mica-oxyde de fer brun vendu sous la référence Pearlescent Pigment Flonac MX 30 C par la société Sudarshan Chemical | 10,50 |
| | TOTAL | 100% |

| Exemples | **Huile** | **Aspect** |
|---|---|---|
| Exemple 13 (Invention) | Dodécaméthylpentasiloxane | Homogénéisation facile des nacres |
| Exemple 14 (Invention) | Octyldodécanol | Homogénéisation facile des nacres |

### Mode opératoire

Les compositions des exemples 13 et 14 sont préparées selon le protocole décrit ci-dessus pour les exemples 1 à 3.

Il a été constaté que le changement de la nature de l'huile selon les exemples 13 et 14 permet d'obtenir des compositions conformes à l'invention.

### Test d'évaluation de l'adhérence des nacres aux parois

Afin d'évaluer l'adhérence des nacres aux parois, le test suivant est effectué dans le cadre de la présente invention :
On introduit un échantillon de 20 g de la composition dans un flacon en verre d'environ 30 mL (diamètre 2,5 cm, hauteur 8cm).

Afin de garantir que l'échantillon prélevé est fidèle à la composition, on le conditionne après avoir homogénéisé la composition sous agitation. On peut ainsi prélever l'échantillon juste après la fabrication.

Le flacon est laissé au repos pendant 24 heures en position verticale, avant d'être évalué.

On juge ensuite la propreté des parois du flacon :
- si l'intégralité des nacres se trouve sédimentée dans le milieu liquide, celle-ci est conforme à l'invention,
- si une partie des nacres est fixée aux parois du flacon, alors la composition n'est pas conforme à l'invention.

### Exemples 15 à 17 : Influence de la nature du substrat de la nacre

Les exemples 15 à 17 permettent de montrer l'influence de la nature du substrat de la nacre (particules (P2)) sur le phénomène d'adhésion aux parois.

| | | **% massique** |
|---|---|---|
| A | Eau | 10,50 |
| | Ethanol absolu | 16,8998 |
| | Propylène glycol | 25,20 |
| | Glycérol | 16,90 |
| | D&C Red N°4 | 0,0001 |
| B | Isononyl isononanoate | 20,00 |
| | D&C Violet N°2 | 0,0001 |
| **C** | **Nacre** | **10,50** |
| | TOTAL | 100% |

### Mode opératoire

On prépare séparément la phase aqueuse A en pesant les constituants dans un bécher puis en agitant au barreau aimanté jusqu'à homogénéisation.

On prépare séparément, de la même manière, la phase huileuse B.

On introduit ensuite la phase aqueuse A dans le bécher principal que l'on agite à l'aide d'une turbine défloculeuse-Raynerie à une vitesse de 500 tours / min.

La nacre (phase C) est ensuite introduite dans la phase aqueuse sous agitation de manière à l'homogénéiser correctement.

Puis la phase huileuse B est introduite dans le bécher en maintenant sous agitation.

On agite encore pendant 15 min la composition avant conditionnement.

### Adhésion des nacres aux parois

| **Exemples** | **Nacres** | **Structure** | **Aspect** |
|---|---|---|---|
| Exemple 15 (invention) (particules (P2)) | Silice- oxyde de titane-oxyde d'étain vendu sous la référence Xirona Magic Mauve par la société Merck | SiO₂/TiO₂/SnO₂ (88/10/2) | Parois propres, nacres sédimentées dans le milieu liquide |
| Exemple 16 (invention) (particules (P2)) | Calcium aluminium borosilicate- oxyde de titane-oxyde d'étain vendu sous la référence Metashine MC1080RR par la société Nippon Sheet Glass | Borosilicate/TiO₂/SnO₂ (78/21,5/0,5) | Parois propres, nacres sédimentées dans le milieu liquide |
| Exemple 17 (invention - particules (P1)) | Mica-oxyde de titane-oxyde de fer brun vendu sous la référence Pearlescent Pigment Flonac MX 30 C par la société Sudarshan Chemical | Mica/TiO₂/Ox.fer (82/14/4) | Présence des nacres aux parois du flacon et dans le milieu liquide |

Les exemples 15 et 16 correspondent aux particules (P2) des exemples 2 et 3 ci-dessus et l'exemple 17 correspond aux particules (P1) de l'exemple 1 ci-dessus.

On a donc constaté que des particules de nacres (P2) comme décrites ci-dessus dans la description permettent d'obtenir une composition conforme à l'invention, c'est-à-dire une composition triphasique au repos. De plus, dans ces compositions, les nacres sont confinées dans le milieu liquide et n'adhèrent pas aux parois du flacon les contenant.

Lorsque le substrat est différent de celui des particules (P2) de l'invention, les compositions ne sont pas aussi satisfaisantes dans la mesure où les particules de nacres adhèrent aux parois du flacon.

Les particules (P1) permettent d'obtenir des compositions triphasiques mais les compositions obtenues sont moins avantageuses en termes de propriétés d'adhésion des nacres aux parois.

### Evaluation sensorielle

Il a été demandé à un panel de 4 femmes, âgées de 25 à 50 ans, de maquiller l'exemple 2 sur le visage et le buste.

Il ressort de cette évaluation que le produit présente une texture fine, glissante et facile à travailler.

### Exemples 18 à 21 : Influence de la position de la couche de silice

Les exemples 18 à 21 permettent de montrer l'influence de la position de la couche de silice de particules (P2) sur le phénomène d'adhésion aux parois.

| | | % massique |
|---|---|---|
| A | Eau | 10,50 |
| | Ethanol absolu | 16,8998 |
| | Propylène glycol | 25,20 |
| | Glycérol | 16,90 |
| | D&C Red N°4 | 0,0001 |
| B | Isononyl isononanoate | 20,00 |
| | D&C Violet N°2 | 0,0001 |
| **C** | **Nacre** | **10,50** |
| | TOTAL | 100% |

### Mode opératoire

Les compositions des exemples 18 à 21 sont préparées selon le protocole décrit ci-dessus pour les exemples 1 à 3.

### Adhésion des nacres aux parois

| **Exemples** | **Nacres** | **Structure** | **Position couche SiO2** | **Aspect** |
|---|---|---|---|---|
| Exemple 18 (Invention) | Mica-oxyde de titane-silice vendu sous la référence Timiron splendid red par la société Merck | Mica/TiO₂/SiO₂ (33/55/12) | Externe | Parois propres, nacres sédimentées dans le milieu liquide |
| Exemple 19 (Invention) | Mica-oxyde de titane-silice vendu sous la référence Timiron artic silver par la société Merck | Mica/TiO₂/SiO₂ (46/40/14) | Externe | Parois propres, nacres sédimentées dans le milieu liquide |
| Exemple 20 (Invention) | Mica-oxyde de titane-Ox. fer-silice- vendu sous la référence Colorona precious gold par la société Merck | Mica/TiO₂/Ox.fer/SiO₂ (35/24/20/20) | Externe | Parois propres, nacres sédimentées dans le milieu liquide |
| Exemple 21 (Comparatif) | Mica-oxyde de titane -Silice-oxyde de titane-oxyde d'étain vendu sous la référence Xirona Glitter red gold par la société Merck | Mica/TiO₂/SiO₂/TiO₂/SnO₂ (62,5/19/17,5/1) | Interne | Présence des nacres aux parois du flacon et dans le milieu liquide |

On a donc constaté que des particules de nacres (P2) comme décrites ci-dessus dans la description (contenant du mica comme substrat et une couche externe de silice) permettent d'obtenir une composition conforme à l'invention, c'est-à-dire une composition triphasique au repos dans laquelle les nacres sont confinées dans le milieu liquide.

Lorsque les particules de nacres (P2) contiennent du mica comme substrat mais pas de couche externe de silice, alors ces particules de nacres adhèrent aux parois du flacon, ce qui n'est pas satisfaisant selon l'invention.

### Exemple 22 : Influence du pourcentage d'oxyde de fer

L'exemple 22, que l'on compare à l'exemple 15 (invention), permet de montrer l'influence du pourcentage d'oxyde de fer qui, selon l'invention, doit être inférieur ou égal à 50%.

| | | **% massique** |
|---|---|---|
| A | Eau | 10,50 |
| | Ethanol absolu | 16,8998 |
| | Propylène glycol | 25,20 |
| | Glycérol | 16,90 |
| | D&C Red N°4 | 0,0001 |
| B | Isononyl isononanoate | 20,00 |
| | D&C Violet N°2 | 0,0001 |
| **C** | **Nacre** | **10,50** |
| | TOTAL | 100% |

### Mode opératoire

Les compositions de l'exemple 22 sont préparées selon le protocole décrit ci-dessus pour les exemples 15 à 17.

### Adhésion des nacres aux parois

| **Exemples** | **Nacres** | **Structure** | **% oxyde de fer** | **Aspect** |
|---|---|---|---|---|
| Exemple 15 (invention) | Silice- oxyde de titane-oxyde d'étain vendu sous la référence Xirona Magic Mauve par la société Merck | SiO2/TiO2/SnO2 (88/10/2) | 0 | Parois propres, nacres sédimentées dans le milieu liquide |
| Exemple 22 (Comparatif) | Silice- oxyde de fer vendu sous la référence Xirona Le Rouge par la société Merck | SiO2/Ox. Fer (45/55) | 55 | Présence des nacres aux parois du flacon et dans le milieu liquide |

On a donc constaté que des particules de nacres (P2) comme décrites ci-dessus dans la description (contenant mois de 50% en poids de d'oxyde de fer) permettent d'obtenir une composition conforme à l'invention, c'est-à-dire une composition triphasique au repos dans laquelle les nacres sont confinées dans le milieu liquide.

Lorsque la teneur en oxyde de fer est supérieure à 50% en poids, les nacres adhèrent aux parois du flacon, ce qui n'est pas satisfaisant selon l'invention.

## Revendications

1. Composition cosmétique comprenant un milieu physiologiquement acceptable contenant :
(1) une phase aqueuse ;
(2) une phase huileuse de densité inférieure à celle de la phase aqueuse ; et
(3) des particules de nacres, dispersées dans la phase aqueuse, constituées d'un substrat enrobé, partiellement ou totalement, d'une ou plusieurs couches, la taille moyenne desdites particules de nacres étant comprise entre 2 µm et 1 000 µm,
ledit substrat étant du mica ou de l'alumine,
l'une au moins des couches étant une couche d'oxyde métallique,
lesdites particules ne comprenant pas de couche de silice en surface, et
sous réserve que, lorsque la couche d'oxyde métallique comprend plus de 30% en poids de dioxyde de titane par rapport au poids total des particules, la taille moyenne desdites particules est comprise entre 2 µm et 20 µm ;
ladite composition cosmétique comprenant de 5% à 70% en poids de phase huileuse par rapport au poids total de ladite composition.

2. Composition cosmétique selon la revendication 1, dans laquelle le substrat des particules est enrobé totalement par au moins une couche d'un autre matériau.

3. Composition cosmétique selon la revendication 1 ou 2, dans laquelle le substrat des particules est du mica, naturel ou synthétique.

4. Composition cosmétique selon l'une quelconque des revendications 1 à 3, comprenant de 0,1% à 30% en poids de particules de nacres par rapport au poids total de ladite composition.

5. Composition cosmétique selon l'une quelconque des revendications 1 à 4, dans laquelle les oxydes métalliques sont choisis dans le groupe constitué de l'oxyde de titane, des oxydes de fer, des oxydes de chrome, des oxydes d'étain, des oxydes d'alumine et de leurs mélanges.

6. Composition cosmétique selon l'une quelconque des revendications 1 à 5, dans laquelle le substrat des particules est enrobé d'une couche unique d'oxyde métallique ou de plusieurs couches distinctes d'oxyde métallique.

7. Composition cosmétique selon l'une quelconque des revendications 1 à 6, dans laquelle les particules comprennent au moins une couche d'oxyde de fer et/ou au moins une couche d'oxyde de titane.

8. Composition cosmétique selon l'une quelconque des revendications 1 à 7, dans laquelle les particules de nacres comprennent en outre au moins une couche constituée d'oxychlorure de bismuth, de bleu d'outremer, de bleu de Prusse, de violet de manganèse, de carmin de cochenille et de leurs mélanges.

9. Composition cosmétique selon l'une quelconque des revendications 1 à 8, dans laquelle la phase aqueuse comprend de 5% à 80% en poids d'eau par rapport au poids total de ladite composition.

10. Composition cosmétique selon l'une quelconque des revendications 1 à 9, dans laquelle la phase aqueuse comprend au moins un solvant organique miscible à l'eau.

11. Composition cosmétique selon l'une quelconque des revendications 1 à 10, dans laquelle la phase huileuse comprend au moins une huile choisie dans le groupe constitué des huiles hydrocarbonées, des huiles siliconées, des huiles fluorées et de leurs mélanges.

12. Composition cosmétique selon l'une quelconque des revendications 1 à 11, présentant au repos trois phases visuellement distinctes.

13. Composition cosmétique selon la revendication 12, dans laquelle, au repos, la phase huileuse constitue la couche supérieure, la phase aqueuse la couche intermédiaire et les nacres la couche inférieure.

14. Procédé non thérapeutique de maquillage et/ou de soin de la peau comprenant une étape d'application sur la peau d'au moins une couche d'une composition selon l'une quelconque des revendications 1 à 13.

15. Procédé de revêtement des fibres kératiniques comprenant une étape d'application sur lesdites fibres kératiniques d'une composition cosmétique selon l'une quelconque des revendications 1 à 13.
